# EUROPÄISCHE PATENTSCHRIFT

(11) **EP 0 518 882 B1**
(45) Veröffentlichungstag und Bekanntmachung des Hinweises auf die Patenterteilung: **27.07.1994**
(21) Anmeldenummer: 91904215.0
(22) Anmeldetag: 27.02.1991
(51) Int. Cl.: C07C 323/41, C07C 319/18

(54) **N-ACYL-AMINOALKYL-2-HYDROXYETHYLSULFIDE UND EIN VERFAHREN ZU IHRER HERSTELLUNG**
N-ACYL-AMINOALKYL-2-HYDROXYETHYLSULPHIDES AND A METHOD FOR PREPARING THEM
N-ACYL-AMINOALKYL-2-HYDROXYETHYLSULFURE ET UN PROCEDE DE PREPARATION

(30) Priorität: 07.03.1990 DE 4007048
(43) Veröffentlichungstag der Anmeldung: 23.12.1992
(73) Patentinhaber: HOECHST AKTIENGESELLSCHAFT, 65926 Frankfurt am Main (DE)
(72) Erfinder: MEIER, Michael, D-6000 Frankfurt am Main 90 (DE); ANGENENDT, Heinrich, D-6000 Frankfurt am Main (DE); MISCHKE, Peter, D-6232 Bad Soden am Taunus (DE)
(86) Internationale Anmeldenummer: EP9100360
(87) Internationale Veröffentlichungsnummer: WO9113867

(56) Entgegenhaltungen:
- DE-A- 2 040 620
- FR-A- 2 447 966
- US-A- 3 849 576

## Beschreibung

Die Erfindung betrifft N-Acyl-aminoalkyl-2-hydroxyethylsulfide der allgemeinen Formel (A)

R-CONH-(CH₂)ₙ-S-CH₂-CH₂-OH (A),

in welcher R ein Wasserstoffatom oder einen Alkyl(C₁-C₄)- Rest und n eine ganze Zahl von 3 bis 6 bedeuten, und ein Verfahren zu ihrer Herstellung durch Umsetzung von Verbindungen der allgemeinen Formel (B)

R-CONH-(CH₂)ₘ-CH=CH₂ (B),

in welcher R die vorstehend genannte Bedeutung hat und m eine ganze Zahl von 1 bis 4 darstellt, mit Mercaptoethanol in Gegenwart eines Radikalstarters.

Die erfindungsgemäßen N-Acyl-aminoalkyl-2-hydroxyethylsulfide sind Vorprodukte zur Herstellung von Aminoalkylethylsulfonen der allgemeinen Formel (C)

H₂N-(CH₂)ₙ-SO₂-CH₂-CH₂-X (C),

in welcher n die vorstehend genannte Bedeutung hat und X einen anorganischen oder organischen Rest, vorzugsweise eine Gruppe bzw. ein Element aus der Reihe -OH, -OSO₃H, -Cl oder OCOCH₃ darstellt, die ihrerseits als Komponenten zur Herstellung von Reaktivfarbstoffen dienen (DE 2 040 620 oder EP 0 141 776).

Die Verbindungen der genannten Formel (C) konnten bisher durch Oxidation der nicht acylierten Aminoalkylsulfide der allgemeinen Formel (D)

H₂N-(CH₂)ₙ-S-CH₂-CH₂-X (D),

in welcher n und X die vorstehend genannten Bedeutungen haben, erhalten werden.

Zur Herstellung der Sulfide der vorstehend genannten Formel (D) sind bereits mehrere Verfahren bekannt. So wird gemäß J. Med. Chem. 9, 217(1966) 3-Aminopropyl-2-hydroxy- ethylsulfid durch Reaktion von Mercaptoethanol mit 3-Bromaminopropanhydrobromid erhalten. Nachteilig an dieser Methode ist jedoch der Anfall von 2 Äquivalenten Natriumbromid. Ein verbessertes, ohne Salzanfall arbeitendes Verfahren zur Herstellung dieses Sulfides wird in DE 2 040 620 beschrieben: Allylamin wird unter Zusatz von Azoisobutyronitril (AIBN) mit Mercaptoethanol bei 50-150°C umgesetzt. Ausbeuten werden hierbei nicht angegeben. Eine Nacharbeitung des Verfahrens (siehe Beispiel 9 weiter unten) ergab lediglich Ausbeuten von 56.6 % d.Th.. Somit ist festzustellen, daß auch diese Herstellungsverfahren den Ansprüchen an ein technisches Verfahren nicht gerecht wird.

Eine Modifikation dieses Herstellungsverfahrens wird durch die Umsetzung von 4-Nitrobenzoylallylamid bei 120-140°C mit Mercaptoethanol, ebenfalls unter Zusatz von Radikalbildnern beschrieben (DE 2 040 620, Beispiel 1). Auch hierbei werden keine Ausbeuten angegeben. Eine Nacharbeitung (siehe Beispiel 11 weiter unten) ergab eine Rohausbeute von 78.3 % d.Th.. Dieses abgewandelte Verfahren ist jedoch auf Grund des Anfalls von 3-Nitrobenzoesäure kein wirtschaftlicher Weg, um 3-Aminopropyl-2-hydroxyethylsulfid herzustellen.

In der US-PS 3 278 526 wird die Umsetzung von N-Alkenylamiden mit Schwefelwasserstoff und Mercaptanen unter Zusatz von Radikalinitiatoren beschrieben, wobei die Ausbeuten mit maximal 66 % unbefriedigend sind. Auch die japanische Patentschrift 44-10770 lehrt, daß bei der Umsetzung von N-Acylalkenylamiden mit Mercaptanen der Zusatz von speziellen Radikalinitiatoren notwendig ist. Die Ausbeuten betragen hierbei 57 - 91 % d.Th..

Aus vorstehend aufgeführten Gründen bestand ein Bedürfnis für ein wirtschaftliches und technisch leicht durchführbares Verfahren zur Herstellung von N-Acylaminoalkyl-2-hydroxyethylsulfiden.

Es wurde nun gefunden, daß man N-Acyl-aminoalkyl-2-hydroxyethylsulfide der allgemeinen Formel (1)

R-CONH-(CH₂)ₙ-S-CH₂-CH₂-OH (1)

in welcher R ein Wasserstoffatom oder einen unverzweigten oder verzweigten Alkyl(C₁-C₄)-Rest und n eine ganze Zahl von 3 bis 6 bedeuten, in hohen Ausbeuten und in hoher Reinheit herstellen kann, indem man Verbindungen der allgemeinen Formel (2)

R-CONH-(CH₂)ₘ-CH=CH₂ (2)

in welcher R die vorstehend genannte Bedeutung hat und m eine ganze Zahl von 1 bis 4 darstellt, mit der äquivalenten Menge Mercaptoethanol bei Temperaturen von etwa 15 bis etwa 150°C, vorzugsweise von etwa 20 bis etwa 100°C, besonders bevorzugt von etwa 25 bis etwa 80°C in Gegenwart eines Radikalstarters in Gegenwart oder in Abwesenheit eines gegenüber den Reaktionsteilnehmern und gegenüber radikalischen Reaktionen inerten Lösungsmittels umsetzt.

Als Radikalbildner dient vorzugsweise Sauerstoff. Dieser kann beispielsweise in reiner Form oder als Mischung mit einem Inertgas oder einem Inertgasgemisch, bevorzugt in Form von Luft, ins Reaktionsgemisch eingebracht werden.

Verfahrensgemäß kann man entweder so vorgehen, daß man beide Reaktionspartner mischt und in Gegenwart von Luft und/oder Sauerstoff auf Reaktionstemperatur bringt, oder so, daß man einen der beiden Reaktionspartner vorlegt und den zweiten Reaktionspartner innerhalb der genannten Temperaturbereiche zudosiert. Dabei ist es im Prinzip unerheblich, ob die Verbindung der genannten Formel (2) vorgelegt und das Mercaptoethanol zudosiert wird oder ob umgekehrt verfahren wird.

Als inertes Lösungsmittel für die Reaktion kann ein ggfs. halogenierter aromatischer Kohlenwasserstoff oder ein Paraffin oder Paraffingemisch oder ein halogenierter aliphatischer Kohlenwasserstoff, der bei der jeweils angewandten Reaktionstemperatur flüssig ist, wie beispielsweise Benzol, Monochlorbenzol, 1,2-, 1,3- oder 1,4-Dichlorbenzol, Trichlorbenzol, Tetrachlorkohlenstoff oder ein Paraffin mit mindestens 6 C-Atomen eingesetzt werden.

Die Reaktion kann bei Normaldruck oder erhöhtem Druck vorgenommen werden. Bevorzugt ist jedoch das Arbeiten bei Normaldruck.

Von den Ausgangsverbindungen der genannten allgemeinen Formel (2) seien beispielsweise das N-Allylacetamid, N-Allylformamid, N-Allylpropionylamid, N-Allylvaleroylamid, N-Butenyl(3)-formamid, N-Butenyl(3)-acetamid, N-Pentenyl(4)-formamid, N-Pentenyl(4)-acetamid, N-Hexenyl(5)-formamid und N-Hexenyl(5)-acetamid genannt.

Wie schon dargelegt, ist es zweckmäßig und optimal, die beiden Reaktionskomponenten in äquivalenten Mengen zur Umsetzung zu bringen. Es ist jedoch auch möglich, jede der beiden Reaktionskomponenten gegenüber der jeweils anderen im Über- bzw. Unterschuß einzusetzen. Eine solche Verfahrensweise ist jedoch nicht als zweckmäßig anzusehen, weil die im Überschuß eingesetzte Komponente anschließend destillativ entfernt werden muß.

Es ist als überraschend zu erachten, daß die Verbindungen der genannten allgemeinen Formel (1) selbst bei Raumtemperatur aus der Verbindung der genannten allgemeinen Formel (2) und Mercaptoethanol in hohen Ausbeuten und in hoher Reinheit gebildet werden und diese Reaktion entgegen dem bisherigen Stand der Technik (siehe Vergleichsbeispiel 7) lediglich durch Sauerstoff und in besonders einfacher Ausführungsform durch Einleiten von Luft, d.h. ohne Verwendung von speziellen Radikalstartern, wie z.B. Azoisobutyronitril, eingeleitet werden kann.

Die erfindungsgemäß erhältlichen Verbindungen der genannten allgemeinen Formel (1) können direkt zur Oxidation zum entsprechenden Sulfon bzw. durch Entacylierung zu den Aminen der eingangs genannten allgemeinen Formel (2) umgesetzt werden, die wiederum als Vorprodukte zur Herstellung von Reaktivfarbstoffen Verwendung finden.

Die nachstehenden Beispiele dienen zur Erläuterung der vorliegenden Erfindung, ohne sie darauf zu beschränken.

### Beispiel 1 (N-Acetyl-3-aminopropyl-2-hydroxyethylsulfid)

Eine Mischung aus 99,1 g (1,0 mol) N-Allylacetamid und 78.1 g (1,0 mol) Mercaptoethanol werden bei 25°C unter Luftzutritt heftig gerührt. Nach 8 h ist die Reaktion beendet (GC). Es fallen 177,1 g N-Acetyl-3-aminopropyl-2-hydroxyethylsulfid als gelbliches Öl mit einer Reinheit nach GC von 94,3 % an, was einer Ausbeute von 94,2 % d.Th. entspricht.
¹H-NMR (CDCl₃): δ = 1.50-1.95 (m;2H;CH₂CH₂CH₂), 1.95 (s;3H; COCH₃), 2.55, 2.62 (t, J=7Hz;4H;2 -CH₂S), 3.25 (pseudo q, J=7Hz;2H;CH₂NH), 3.70 (t, J=7Hz;2H;CH₂OH), 4.3 (s;1H;OH), 7.1-7.6 (m;1H;NH).
IR (Film): 3310 (NH,OH), 3095 (NH), 2950 (CH₂), 2870 (CH₂), 1645 (CO), 1560 (CO).
MS : 178 (M⁺+1), 159 (M⁺-H₂O), 100 (M⁺-SCH₂CH₂OH).

### Beispiel 2 (N-Acetyl-3-aminopropyl-2-hydroxyethylsulfid)

Zu 99,1 g (1,0 mol) N-Allylacetamid in 250 ml Chlorbenzol tropft man bei 60°C über 2 h 78,1 g (1,0 mol) Mercaptoethanol in 100 ml Chlorbenzol und rührt 10 h unter Luftzutritt nach. Nach dem Abdestillieren des Lösungsmittels erhält man 175,8 g Acetyl-1-aminopropyl-2-hydroxyethylsulfid mit einer Reinheit von 95,5 %, was einer Ausbeute von 94,7 % d.Th. entspricht.

Die spektroskopischen Daten, betreffend Kernresonanz-, Massen- und Infrarotspektrum, sind mit denen, die in Beispiel 1 genannt sind, identisch.

### Beispiel 3 (N-Acetyl-3-aminopropyl-2-hydroxyethylsulfid)

Zu 78,1 g (1,0 mol) Mercaptoethanol werden unter starkem Rühren unter Luftzutritt bei 25°C über 1 h 99,1 g (1,0 mol)

N-Allylacetamid getropft. Nach Nachrühren über 7 h bei Raumtemperatur fallen 177,2 g 3-Acetylaminopropyl-2-hydroxyethylsulfid mit einer Reinheit von 95,7 % an, was einer Ausbeute von 95,7 % d.Th. entspricht.

Die spektroskopischen Daten, betreffend Kernresonanz-, Massen- und Infrarotspektrum, sind mit denen, die in Beispiel 1 genannt sind, identisch.

### Beispiel 4 (N-Formyl-3-aminopropyl-2-hydroxyethylsulfid)

Zu 85,1 g (1,0 mol) N-Allylformamid werden unter starkem Rühren unter Luftzutritt bei 25°C über 1 h 78,1 g (1,0 mol) Mercaptoethanol getropft. Nach Nachrühren über 5 h bei Raumtemperatur fallen 163,2 g N-Formyl-3-aminopropyl-2-hydroxyethylsulfid mit einer Reinheit von 95,6 % an, was einer Ausbeute von 95,6 % d.Th. entspricht.
¹H-NMR (CDCl₃): δ = 1.30-2.10 (m;2H;CH₂CH₂CH₂), 2.58, 2.68 (t, J=7Hz;4H;2 -CH₂S), 3.35 (pseudo q, J=7Hz;2H;CH₂NH), 3.72 (t, J=7Hz;2H;CH₂OH), 4.35 (s;1H;OH), 7.1-7.7 (m;1H;NH), 8.05 (d, J=14Hz;O,2H;z-CHO), 8.10 (d, J=2Hz; 0,8H;e-CHO).
IR (Film): 3310 (NH,OH), 3080 (NH), 2940 (CH₂), 2880 (CH₂), 1670 (CO), 1540 (CO).
MS : 145 (M⁺-H₂O), 86 (M⁺-SCH₂CH₂OH).

### Beispiel 5 (N-Valeroyl-3-aminopropyl-2-hydroxyethylsulfid)

Zu 142,1 g (1,0 mol) N-Allylvaleroylamid werden unter starkem Rühren unter Luftzutritt bei 25°C über 2 h 78,1 g (1,0 mol) Mercaptoethanol getropft. Nach Nachrühren über 7 h bei Raumtemperatur fallen 219,2 g 3-Valeroylaminopropyl-2-hydroxyethylsulfid mit einer Reinheit von 96,2 % an, was einer Ausbeute von 96,2 % d.Th. entspricht.
Der Schmelzpunkt beträgt 7°C.
¹H-NMR (CDCl₃): δ = 0.70-2.30 (m;11H), 2.35, 2.62 (t, J=7Hz;4H;2 -CH₂S), 3.25 (pseudo q, J=7Hz;2;CH₂NH), 3.70 (t, J=7Hz;2H;CH₂OH), 4.9 (s;1H;OH), 6.9-7.5 (m;1H;NH).
IR (Film): 3300 (NH,OH), 3090 (NH), 2940 (CH₂), 2880 (CH₂), 1650 (CO), 1550 (CO).
MS : 219 (M⁺), 201 (M⁺-H₂O), 142 (M⁺-SCH₂CH₂OH).

### Beispiel 6 (N-Acetyl-3-aminopropyl-2-hydroxyethylsulfid)

In eine Mischung aus 99,1 g (1,0 mol) N-Allylacetamid und 78,1 g (1,0 mol) Mercaptoethanol wird bei 25°C ein Sauerstoff/Stickstoffgemisch (1:5) unter heftigem Rühren eingeleitet. Nach 8 h ist die Reaktion beendet (GC). Es fallen 173,4 g N-Acetyl-3-aminopropyl-2-hydroxyethylsulfid als gelbliches Öl mit einer Reinheit nach GC von 95,6 % an, was einer Ausbeute von 93,5 % d.Th. entspricht.

Die spektroskopischen Daten, betreffend Kernresonanz- und Infrarotspektrum, sind mit denen, die in Beispiel 1 genannt sind, identisch.

### Beispiel 7 (N-Acetyl-3-aminopropyl-2-hydroxyethylsulfid)

Zu 99,1 g (1,0 mol) N-Allylacetamid tropft man bei 80°C über 2 h 78,1 g (1,0 mol) Mercaptoethanol unter Luftzutritt zu und rührt 4 h nach. Man erhält 177,1 g Acetyl-3-aminopropyl-2-hydroxyethylsulfid mit einer Reinheit von 91,5 %, was einer Ausbeute von 91,5 % d.Th. entspricht.

Die spektroskopischen Daten, betreffend Kernresonanz-, Massen- und Infrarotspektrum, sind mit denen, die in Beispiel 1 genannt sind, identisch.

### Beispiel 8 (Vergleichsbeispiel)

Eine Mischung aus 99,1 g (1,0 mol) N-Allylacetamid und 78,1 g (1,0 mol) Mercaptoethanol wird bei 25°C unter Stickstoffatmosphäre heftig gerührt. Nach 8 h hat sich nach GC kein Reaktionsprodukt gebildet und die Ausgangsmaterialien liegen unverändert vor.

### Beispiel 9 (3-Aminopropyl-2-hydroxyethylsulfid)

- Vergleichsbeispiel nach Angaben von Beispiel 3, Seite 18 von DE 2 040 620 -

Zu 78,1 g (1,0 mol) Mercaptoethanol, das mit 0,5 g Azoisobutyronitril versetzt ist, werden bei 80-90°C über 2 h 57,0 g (1,0 mol) Allylamin getropft. Anschließend wird bei 10 h bei 80-90°C nachgerührt. Es fallen 126,8 g 3-Aminopropyl-2-hydroxyethylsulfid mit einer Reinheit von 60,4 % an, was einer Ausbeute von 56,6 % d.Th. entspricht.

### Beispiel 10 (3-Aminopropyl-2-hydroxyethylsulfid)

- Vergleichsbeispiel nach Angaben von Beispiel 3, Seite 18 von DE 2 040 620, wobei jedoch als Radikalstarter Luft verwendet wurde -

78,1 g (1,0 mol) Mercaptoethanol und 57,0 g (1,0 mol)

Allylamin werden bei 60°C heftig gerührt. Es fallen 124,7 g Reaktionsgemisch an, das laut GC und GC-MS zu 12 % aus 3-Aminopropyl-2-hydroxyethylsulfid, 19 % aus Bis(2-hydroxyethylsulfid) und nicht umgesetzten Ausgangsmaterialien besteht.

### Beispiel 11 (N-4-Nitrobenzoyl-3-aminopropyl-2-hydroxy-ethylsulfid)

- Vergleichsbeispiel nach Angaben von Beispiel 1, Seite 15, von DE 2 040 620 -

Zu 103 g (0,5 mol) 4-Nitrobenzoylallylamid werden in der Schmelze bei 120-140°C, welcher 0,1 g Azoisobutyronitril zugesetzt wurden, 40 g (0,5 mol) Mercaptoethanol gegeben. Dann wird 5 h bei dieser Temperatur nachgerührt. Es fallen 143 g N-4-Nitrobenzoyl-3-aminopropyl-2-hydroxyethylsulfid mit einer Reinheit von 78,3 % an, was einer Ausbeute von 78,3 % d.Th. entspricht.

## Patentansprüche

1. N-Acyl-aminoalkyl-2-hydroxyethylsulfide der allgemeinen Formel
R-CONH-(CH₂)ₙ-S-CH₂-CH₂-OH,
in welcher R ein Wasserstoffatom oder einen unverzweigten oder verzweigten Alkyl(C₁-C₄)-Rest und n eine ganze Zahl von 3 bis 6 bedeuten.

2. Die Verbindung der Formel
CH₃-CONH-CH₂-CH₂-CH₂-S-CH₂-CH₂-OH .

3. Die Verbindung der Formel
HCO-NH-CH₂-CH₂-CH₂-S-CH₂-CH₂-OH .

4. Die Verbindung der Formel
C₄H₉-CO-NH-CH₂-CH₂-CH₂-S-CH₂-CH₂-OH .

5. Die Verbindung der Formel
C₃H₇-CONH-CH₂-CH₂-CH₂-S-CH₂-CH₂-OH .

6. Verfahren zur Herstellung von N-Acyl-aminoalkyl-2-hydroxyethylsulfiden der allgemeinen Formel (1)
R-CONH-(CH₂)ₙ-S-CH₂-CH₂-OH (1),
in welcher R ein Wasserstoffatom oder einen unverzweigten oder verzweigten Alkyl(C₁-C₄)-Rest und n eine ganze Zahl von 3 bis 6 bedeuten, dadurch gekennzeichnet, daß man Verbindungen der allgemeinen Formel (2)
R-CONH-(CH₂)ₘ-CH=CH₂ (2),
in welcher R die vorstehend genannte Bedeutung hat und m eine ganze Zahl von 1 bis 4 darstellt, mit der äquivalenten Menge Mercaptoethanol bei Temperaturen von etwa 15 bis etwa 150°C in Gegenwart eines Radikalstarters in Gegenwart oder in Abwesenheit eines gegenüber den Reaktionsteilnehmern und gegenüber radikalischen Reaktionen inerten Lösungsmittels umsetzt.

7. Verfahren nach Anspruch 6, dadurch gekennzeichnet, daß man bei Temperaturen von etwa 20 bis etwa 100°C umsetzt.

8. Verfahren nach Anspruch 6, dadurch gekennzeichnet, daß man bei Temperaturen von etwa 25 bis etwa 80°C umsetzt.

9. Verfahren nach mindestens einem der Ansprüche 6 bis 8, dadurch gekennzeichnet, daß man in Gegenwart von Sauerstoff als Radikalstarter umsetzt.

10. Verfahren nach mindestens einem der Ansprüche 6 bis 9, dadurch gekennzeichnet, daß man in Gegenwart einer Mischung aus Sauerstoff und einem Inertgas oder einem Inertgasgemisch als Radikalstarter umsetzt.

11. Verfahren nach mindestens einem der Ansprüche 6 bis 10, dadurch gekennzeichnet, daß man in Gegenwart von Luft als Radikalstarter umsetzt.

12. Verfahren nach mindestens einem der Ansprüche 6 bis 11, dadurch gekennzeichnet, daß man in einem ggfs. halogeinierten aromatischen Kohlenwasserstoff oder in einem Paraffin oder Paraffingemisch oder in einem halogenierten aliphatischen Kohlenwasserstoff als inertem Lösungsmittel umsetzt.

13. Verfahren nach mindestens einem der Ansprüche 6 bis 12, dadurch gekennzeichnet, daß man in Benzol, Monochlorbenzol, 1,2-, 1,3- oder 1,4-Dichlorbenzol, Trichlorbenzol, Tetrachlorkohlenstoff oder in einem Paraffin mit mindestens 6 C-Atomen umsetzt.

14. Verfahren nach mindestens einem der Ansprüche 6 bis 13, dadurch gekennzeichnet, daß man einen der beiden Reaktionspartner vorlegt und den anderen zudosiert.

15. Verfahren nach mindestens einem der Ansprüche 6 bis 14, dadurch gekennzeichnet, daß man bei Normaldruck oder bei erhöhtem Druck umsetzt.

## Claims

1. An N-acylaminoalkyl 2-hydroxyethyl sulfide of the formula
R-CONH-(CH₂)ₙ-S-CH₂-CH₂-OH
in which R is a hydrogen atom or an unbranched or branched alkyl(C₁-C₄) radical and n is an integer from 3 to 6.

2. The compound of the formula
CH₃-CONH-CH₂-CH₂-CH₂-S-CH₂-CH₂-OH.

3. The compound of the formula
HCO-NH-CH₂-CH₂-CH₂-S-CH₂-CH₂-OH.

4. The compound of the formula
C₄H₉-CO-NH-CH₂-CH₂-CH₂-S-CH₂-CH₂-OH.

5. The compound of the formula
C₃H₇-CONH-CH₂-CH₂-CH₂-S-CH₂-CH₂-OH.

6. A process for the preparation of an N-acylaminoalkyl 2-hydroxyethyl sulfide of the formula (1)
R-CONH-(CH₂)ₙ-S-CH₂-CH₂-OH (1)
in which R is a hydrogen atom or an unbranched or branched alkyl(C₁-C₄) radical and n is an integer from 3 to 6, which comprises reacting a compound of the formula (2)
R-CONH-(CH₂)ₘ-CH=CH₂ (2),
in which R is as defined above and m is an integer from 1 to 4, with the equivalent amount of mercaptoethanol at temperatures of about 15 to about 150°C in the presence of a free-radical initiator in the presence or in the absence of a solvent which is inert towards the reactants and towards free-radical reactions.

7. The process as claimed in claim 6, wherein the reaction is carried out at temperatures of about 20 to about 100°C.

8. The process as claimed in claim 6, wherein the reaction is carried out at temperatures of about 25 to about 80°C.

9. The process as claimed in at least one of claims 6 to 8, wherein the reaction is carried out in the presence of oxygen as the free-radical initiator.

10. The process as claimed in at least one of claims 6 to 9, wherein the reaction is carried out in the presence of a mixture of oxygen and an inert gas or an inert gas mixture as the free-radical initiator.

11. The process as claimed in at least one of claims 6 to 10, wherein the reaction is carried out in the presence of air as the free-radical initiator.

12. The process as claimed in at least one of claims 6 to 11, wherein the reaction is carried out in an aromatic hydrocarbon which may be halogenated or in a paraffin or paraffin mixture or in a halogenated aliphatic hydrocarbon as the inert solvent.

13. The process as claimed in at least one of claims 6 to 12, wherein the reaction is carried out in benzene, monochlorobenzene, 1,2-, 1,3- or 1,4-dichlorobenzene, trichlorobenzene, carbon tetrachloride or in a paraffin having at least 6 carbon atoms.

14. The process as claimed in at least one of claims 6 to 13, wherein one of the two reactants is initially introduced and the other is metered in.

15. The process as claimed in at least one of claims 6 to 14, wherein the reaction is carried out at atmospheric pressure or at superatmospheric pressure.

## Revendications

1. Sulfures de N-acyl-aminoalkyl-2-hydroxyéthyle de formule générale
R-CONH-(CH₂)ₙ-S-CH₂-CH₂-OH,
dans laquelle R représente un atome d'hydrogène ou un radical alkyle en (C₁-C₄) linéaire ou ramifié et n représente un nombre entier de 3 à 6.

2. Composé de formule
CH₃-CONH-CH₂-CH₂-CH₂-S-CH₂-CH₂-OH

3. Composé de formule
HCO-NH-CH₂-CH₂-CH₂-S-CH₂-CH₂-OH .

4. Composé de formule
C₄H₉-CO-NH-CH₂-CH₂-CH₂-S-CH₂-CH₂-OH .

5. Composé de formule
C₃H₇-CONH-CH₂-CH₂-CH₂-S-CH₂-CH₂-OH .

6. Procédé pour la préparation de sulfures de N-acylaminoalkyl-2-hydroxyéthyle de formule générale (1)
R-CONH-(CH₂)ₙ-S-CH₂-CH₂-OH (1),
dans laquelle R représente un atome d'hydrogène ou un radical alkyle en (C₁-C₄) linéaire ou ramifié et n représente un nombre entier de 3 à 6, caractérisé en ce qu'on fait réagir des composés de formule générale (2)
R-CONH-(CH₂)ₘ-CH=CH₂ (2),
dans laquelle R a la signification donnée ci-dessus et m représente un nombre entier de 1 à 4, avec la quantité équivalente de mercaptoéthanol à des températures d'environ 15 à environ 150°C en présence d'un amorceur radicalaire en présence ou en l'absence d'un solvant inerte vis-à-vis des participants de la réaction et vis-à-vis des réactions radicalaires.

7. Procédé selon la revendication 6, caractérisé en ce qu'on met en oeuvre la réaction à des températures d'environ 20 à environ 100°C.

8. Procédé selon la revendication 6, caractérisé en ce qu'on met en oeuvre la réaction à des températures d'environ 25 à environ 80°C.

9. Procédé selon au moins l'une des revendications 6 à 8, caractérisé en ce qu'on met en oeuvre la réaction en présence de l'oxygène en tant qu'amorceur radicalaire.

10. Procédé selon au moins l'une des revendications 6 à 9, caractérisé en ce qu'on met en oeuvre la réaction en présence d'un mélange composé d'oxygène et d'un gaz inerte ou d'un mélange de gaz inertes en tant qu'amorceur radicalaire.

11. Procédé selon au moins l'une des revendications 6 à 10, caractérisé en ce qu'on met en oeuvre la réaction en présence de l'air, en tant qu'amorceur radicalaire.

12. Procédé selon au moins l'une des revendications 6 à 11, caractérisé en ce qu'on met en oeuvre la réaction dans un hydrocarbone aromatique éventuellement halogéné ou dans une paraffine ou mélange de paraffine ou dans un hydrocarbone aliphatique halogéné en tant que solvant inerte.

13. Procédé selon au moins l'une des revendications 6 à 12, caractérisé en ce qu'on met en oeuvre la réaction dans le benzène, le monochlorobenzène, le 1,2-, 1,3- ou 1,4-dichlorobenzène, le trichlorobenzène, le tétrachlorocarbone ou une paraffine comportant au moins 6 atomes de carbone.

14. Procédé selon au moins l'une des revendications 6 à 13, caractérisé en ce qu'on introduit l'un des deux partenaires de réaction et on ajoute progressivement l'autre.

15. Procédé selon au moins l'une des revendications 6 à 14, caractérisé en ce qu'on met en oeuvre la réaction à pression normale ou à une pression élevée.
